# FASCICULE DE BREVET EUROPEEN

(11) **EP 3 666 367 B1**
(45) Date de publication et mention de la délivrance du brevet: **07.05.2025**
(21) Numéro de dépôt: 19214494.7
(22) Date de dépôt: 09.12.2019
(51) Int. Cl.: B01D 53/22, C10L 3/10

(54) **INSTALLATION ET PROCÉDÉ DE TRAITEMENT PAR PERMÉATION MEMBRANAIRE D'UN COURANT GAZEUX AVEC AJUSTEMENT DE LA PRESSION D'ASPIRATION DU SECOND PERMÉAT**
ANLAGE UND VERFAHREN ZUR BEHANDLUNG DURCH MEMBRANPERMEATION EINES GASSTROMS MIT ANPASSUNG DES ASPIRATIONSDRUCKS DES ZWEITEN PERMEATS
SYSTEM AND METHOD FOR TREATING A GASEOUS CURRENT BY MEMBRANE PERMEATION WITH ADJUSTMENT OF THE SUCTION PRESSURE OF THE SECOND PERMEATE

(30) Priorité: 14.12.2018 FR 1872937
(43) Date de publication de la demande: 17.06.2020
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BARRAUD, François, 38360 Sassenage (FR); CHAREYRE, Jean-Marc, 38360 Sassenage (FR)
(74) Mandataire: Air Liquide

(56) Documents cités:
- EP-A1- 1 372 822
- WO-A1-2014/121964
- FR-A1- 3 010 640
- US-A- 4 386 944
- US-A- 5 071 451
- US-A1- 2015 336 046
- US-A1- 2018 223 205

## Description

La présente invention est relative à une installation de traitement par perméation membranaire d'un courant gazeux contenant au moins du méthane et du dioxyde de carbone pour produire un courant gazeux riche en méthane - dont la teneur en méthane est conforme aux besoins de son utilisation et à un procédé de commande d'une telle installation.

Elle concerne en particulier l'épuration de biogaz, dans le but de produire du biométhane conforme aux spécifications pour injection dans un réseau de gaz naturel.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH4) et du dioxyde de carbone (CO2) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO2, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL).

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs oeuvrant sur un territoire (agriculteurs, industriels, pouvoirs publics), la production de biométhane aide les territoires à acquérir une plus grande autonomie énergétique.

Plusieurs étapes doivent être franchies entre la collecte du biogaz et l'obtention du biométhane, produit final apte à être comprimé ou liquéfié.

En particulier, plusieurs étapes sont nécessaires avant le traitement qui vise à séparer le dioxyde de carbone pour produire un courant de méthane purifié. Une première étape consiste à comprimer le biogaz qui a été produit et acheminé à pression atmosphérique, cette compression peut être obtenue - de façon classique - via un compresseur. Les étapes suivantes visent à débarrasser le biogaz des composants corrosifs que sont le sulfure d'hydrogène et les composés organiques volatils (COV), les technologies utilisées sont de façon classique l'adsorption à pression modulée (PSA) et le piégeage sur charbon actif. Vient ensuite l'étape qui consiste à séparer le dioxyde de carbone pour disposer in fine de méthane à la pureté requise pour son usage ultérieur.

Le dioxyde de carbone est un contaminant typiquement présent dans le gaz naturel dont il est courant de devoir le débarrasser. Des technologies variées sont utilisées pour cela en fonction des situations ; parmi celles-ci, la technologie membranaire est particulièrement performante lorsque la teneur en CO2 est élevée ; elle est donc pour séparer le CO2 présent dans le biogaz, provenant des gaz de décharge ou des digesteurs de déchets végétaux ou animaux. Les procédés membranaires de séparation de gaz utilisés pour la purification d'un gaz, qu'ils utilisent un ou plusieurs étages de membranes doivent permettre la production d'un gaz à la qualité requise, pour un faible coût, tout en minimisant les pertes du gaz que l'on souhaite valoriser. Ainsi, dans le cas de l'épuration du biogaz, la séparation effectuée est principalement une séparation CH4/CO2, devant permettre la production d'un gaz contenant en fonction de son utilisation plus de 85% de CH4, de préférence plus de 95% de CH4, plus préférentiellement plus de 97,5% de CH4, tout en minimisant les pertes de CH4 dans le gaz résiduaire et le coût d'épuration, ce dernier étant pour une part importante lié à la consommation électrique du dispositif de compression du gaz en amont des membranes.

Le document publié sous le numéro US 2018/223205 A1 décrit un procédé de traitement de traitement du biogaz contenant du H₂S et du CO₂ par élimination du H2S à l'aide de PTSA (« pressure temperature swing adsorption » en anglais) et du CO₂ à l'aide de membranes de séparation des gaz en deux étages.

Le document publié sous le numéro WO2014/121964 A1 décrit un procédé de séparation d'un mélange de gaz d'alimentation comprenant un gaz A et un gaz B à l'aide d'une unité de séparation à membrane pour obtenir un rétentat riche en gaz A dans lequel le côté perméat de la membrane est balayé par un flux de gaz comprenant le gaz B à un pourcentage inférieur à celui du gaz d'alimentation ou ne comprenant pas du tout le gaz B.

Le document publié sous le numéro US 2015/0336046 A1 décrit un procédé de contrôle d'une installation de séparation de gaz comprenant trois étages de séparation membranaire capable de fournir simultanément deux produits ou plus d'une grande pureté.

Il est préférable que le réseau de gaz naturel reçoive un courant de méthane présentant une concentration en méthane constante de manière à ce que les équipements qui utilisent le biométhane aient un fonctionnement régulier.

Partant de là, un problème qui se pose est de fournir une installation permettant l'obtention d'un courant de méthane à concentration constante.

Une solution de la présente invention est une installation pour le traitement par perméation membranaire d'un flux gazeux d'alimentation comprenant au moins du méthane et du dioxyde de carbone, comprenant:
- un compresseur A permettant de comprimer le flux gazeux d'alimentation,
- une première unité de séparation par membrane apte à recevoir le flux gazeux issu du compresseur et à fournir un premier perméat et un premier retentât,
- une seconde unité de séparation par membrane apte à recevoir le premier retentât et à fournir un second perméat et un second retentât, le second perméat étant configuré pour être recyclé dans le flux gazeux d'alimentation en aval du compresseur A,
- une troisième unité de séparation par membrane apte à recevoir le premier perméat et à fournir un troisième perméat et un troisième rétentat, le troisième retentât étant configuré pour être recyclé au compresseur A permettant de comprimer le flux gazeux d'alimentation,

avec chaque unité de séparation par membrane comprenant au moins une membrane plus perméable au dioxyde de carbone qu'au méthane,
   - au moins un moyen de mesure de la pression d'aspiration du second perméat de la seconde unité membranaire,
   - au moins un compresseur B permettant l'aspiration du second perméat et l'ajustement de la pression d'aspiration du second perméat en fonction de la pression d'aspiration mesurée avant de recycler le second perméat dans le flux gazeux d'alimentation en aval du compresseur A,
   - des moyens de transmission de données configurés pour réaliser automatiquement la mesure de la pression d'aspiration du second perméat, la comparaison de cette mesure avec une valeur consigne et l'ajustement de la pression d'aspiration du second perméat par le compresseur B,
   - au moins un moyen de mesure de la concentration en CH4 dans le second rétentat,
   - au moins un moyen d'ajustement de la pression du flux gazeux d'alimentation en fonction de la concentration en CH4 mesurée,
le moyen d'ajustement de la pression du flux gazeux d'alimentation étant le compresseur A ou une vanne de coupure et de mise en pression progressive, et
   - des moyens de transmission de données et de traitement de données configurés pour réaliser automatiquement la comparaison de la mesure de la concentration en CH4 dans le second retentât avec une valeur consigne et l'ajustement de la pression du flux gazeux d'alimentation.

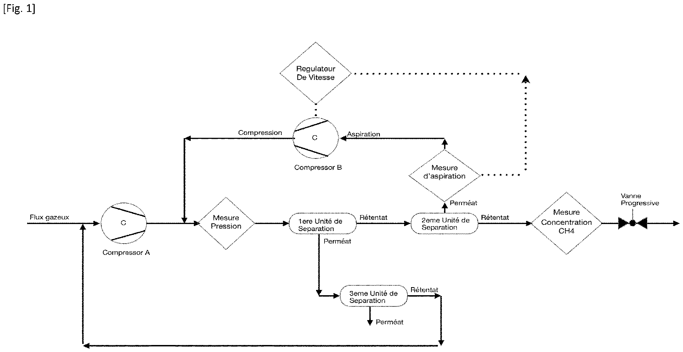

La figure 1 représente un exemple d'installation selon l'invention.

Selon le cas l'installation selon l'invention peut présenter la caractéristique suivante :
- les membranes utilisées dans les unités de séparation par membrane ont la même sélectivité.

La présente invention a également pour objet un procédé de commande d'une installation telle que définie dans l'invention, comprenant les étapes suivantes :
- une étape de mesure de la pression d'aspiration du second perméat,
- une étape de comparaison de cette mesure avec une valeur consigne fixe
- une étape d'ajustement de la pression d'aspiration du second perméat par le compresseur B pour garder la valeur de pression égale à la valeur de consigne,
- une étape de mesure de la concentration en CH4 dans le second retentât,
- une étape de comparaison de cette mesure avec une valeur consigne, et de détermination de l'écart par rapport à cette valeur consigne, et
- une étape d'ajustement de la pression du flux gazeux d'alimentation de manière à réduire l'écart déterminé,
l'ajustement de la pression du flux de gaz d'alimentation étant effectué à l'aide du compresseur A ou à l'aide d'une vanne de coupure et de mise en pression progressive.

Selon le cas, le procédé selon l'invention peut présenter une ou plusieurs des caractéristiques ci-dessous :
- à l'étape d'ajustement le compresseur B subit une accélération ou une décélération ; notons qu'une accélération du compresseur B conduira à une diminution du niveau de pression dans les membranes, et une décélération du compresseur B conduira à une augmentation du niveau de pression dans les membranes;
- les étapes de mesure de la pression d'aspiration du second perméat, de comparaison de cette mesure avec une valeur consigne et d'ajustement de la pression d'aspiration du second perméat sont réalisées automatiquement par des moyens de transmission de données ;
- l'étape d'ajustement du flux gazeux d'alimentation comprend une augmentation ou une diminution de la pression ;
- l'étape de comparaison de la mesure de la concentration en CH4 dans le second retentât avec une valeur consigne et l'étape d'ajustement de la pression du flux gazeux d'alimentation sont réalisées automatiquement par des moyens de transmission de données et de traitement de données ;
- le flux gazeux d'alimentation est du biogaz.

Un moyen de transmission de données et de traitement de données pourra être par exemple un calculateur industriel de type Automate Programmable.

## Revendications

1. Installation pour le traitement par perméation membranaire d'un flux gazeux d'alimentation comprenant au moins du méthane et du dioxyde de carbone, comprenant:
- un compresseur A permettant de comprimer le flux gazeux d'alimentation,
- une première unité de séparation par membrane apte à recevoir le flux gazeux issu du compresseur et à fournir un premier perméat et un premier retentât,
- une seconde unité de séparation par membrane apte à recevoir le premier retentât et à fournir un second perméat et un second retentât, le second perméat étant configuré pour être recyclé dans le flux gazeux d'alimentation en aval du compresseur A,
- une troisième unité de séparation par membrane apte à recevoir le premier perméat et à fournir un troisième perméat et un troisième retentât, le troisième retentât étant configuré pour être recyclé au compresseur A permettant de comprimer le flux gazeux d'alimentation,
avec chaque unité de séparation par membrane comprenant au moins une membrane plus perméable au dioxyde de carbone qu'au méthane,
- au moins un moyen de mesure de la pression d'aspiration du second perméat de la seconde unité membranaire,
- au moins un compresseur B permettant l'aspiration du second perméat et l'ajustement de la pression d'aspiration du second perméat en fonction de la pression d'aspiration mesurée avant de recycler le second perméat dans le flux gazeux d'alimentation en aval du compresseur A,
- des moyens de transmission de données configurés pour réaliser automatiquement la mesure de la pression d'aspiration du second perméat, la comparaison de cette mesure avec une valeur consigne et l'ajustement de la pression d'aspiration du second perméat par le compresseur B,
- au moins un moyen de mesure de la concentration en CH4 dans le second retentât,
- au moins un moyen d'ajustement de la pression du flux gazeux d'alimentation en fonction de la concentration en CH4 mesurée,
le moyen d'ajustement de la pression du flux gazeux d'alimentation étant le compresseur A ou une vanne de coupure et de mise en pression progressive, et
- des moyens de transmission de données et de traitement de données configurés pour réaliser automatiquement la comparaison de la mesure de la concentration en CH4 dans le second retentât avec une valeur consigne et l'ajustement de la pression du flux gazeux d'alimentation.

2. Installation selon la revendication 1, **caractérisée en ce que** les membranes utilisées dans les unités de séparation par membrane ont la même sélectivité.

3. Procédé de commande d'une installation telle que définie dans l'une des revendications 1 ou 2, comprenant les étapes suivantes :
- une étape de mesure de la pression d'aspiration du second perméat,
- une étape de comparaison de cette mesure avec une valeur consigne, et
- une étape d'ajustement de la pression d'aspiration du second perméat par le compresseur B pour garder la valeur de pression égale à la valeur de consigne,
- une étape de mesure de la concentration en CH4 dans le second retentât,
- une étape de comparaison de cette mesure avec une valeur consigne, et de détermination de l'écart par rapport à cette valeur consigne, et
- une étape d'ajustement de la pression du flux gazeux d'alimentation de manière à réduire l'écart déterminé,
l'ajustement de la pression du flux de gaz d'alimentation étant effectué à l'aide du compresseur A ou à l'aide d'une vanne de coupure et de mise en pression progressive.

4. Procédé selon la revendication 3, **caractérisé en ce qu'**à l'étape d'ajustement le compresseur B subit une accélération ou une décélération.

5. Procédé selon l'une des revendications 3 ou 4, **caractérisé en ce que** les étapes de mesure de la pression d'aspiration du second perméat, de comparaison de cette mesure avec une valeur consigne et d'ajustement de la pression d'aspiration du second perméat sont réalisées automatiquement par des moyens de transmission de données.

6. Procédé selon l'une des revendications 3 à 5, **caractérisé en ce que** l'étape d'ajustement du flux gazeux d'alimentation comprend une augmentation ou une diminution de la pression.

7. Procédé selon l'une des revendications 3 à 6, **caractérisé en ce que** l'étape de comparaison de la mesure de la concentration en CH4 dans le second retentât avec une valeur consigne et l'étape d'ajustement de la pression du flux gazeux d'alimentation sont réalisées automatiquement par des moyens de transmission de données et de traitement de données.

8. Procédé selon l'une des revendications 3 à 7, **caractérisé en ce que** le flux gazeux d'alimentation est du biogaz.

## Patentansprüche

1. Anlage zur Behandlung eines Speisegasstroms, der mindestens Methan und Kohlendioxid enthält, durch Membranpermeation, umfassend:
- einen Kompressor A, mit dem der Speisegasstrom komprimiert werden kann,
- eine erste Membrantrenneinheit, die geeignet ist, den Gasstrom aus dem Kompressor aufzunehmen und ein erstes Permeat und ein erstes Retentat zu liefern,
- eine zweite Membrantrenneinheit, die geeignet ist, das erste Retentat aufzunehmen und ein zweites Permeat und ein zweites Retentat bereitzustellen, wobei das zweite Permeat so konfiguriert ist, dass es in den Speisegasstrom stromabwärts des Kompressors A zurückgeführt wird,
- eine dritte Membrantrenneinheit, die geeignet ist, das erste Permeat aufzunehmen und ein drittes Permeat und ein drittes Retentat bereitzustellen, wobei das dritte Retentat so konfiguriert ist, dass es zum Kompressor A zurückgeführt wird, der es ermöglicht, den Speisegasstrom zu komprimieren,
mit jeder Membrantrenneinheit, die mindestens eine Membran umfasst, die für Kohlendioxid durchlässiger ist als für Methan,
- mindestens ein Mittel zur Messung des Saugdrucks des zweiten Permeats aus der zweiten Membraneinheit,
- mindestens einen Kompressor B, der das Ansaugen des zweiten Permeats und die Einstellung des Ansaugdrucks des zweiten Permeats in Abhängigkeit von dem gemessenen Ansaugdruck ermöglicht, bevor das zweite Permeat in den Zufuhrgasstrom stromabwärts des Kompressors A zurückgeführt wird,
- Datenübertragungsmittel, die so konfiguriert sind, dass sie automatisch die Messung des Ansaugdrucks des zweiten Permeats, den Vergleich dieser Messung mit einem Sollwert und die Einstellung des Ansaugdrucks des zweiten Permeats durch den Kompressor B durchführen,
- mindestens ein Mittel zum Messen der CH4-Konzentration in dem zweiten Retentat,
- mindestens ein Mittel zur Einstellung des Drucks des Speisegasstroms in Abhängigkeit von der gemessenen CH4-Konzentration,
das Mittel zum Einstellen des Drucks des Speisegasstroms der Kompressor A oder ein Ventil zur schrittweisen Abschaltung und Druckbeaufschlagung ist und
- Datenübertragungs- und Datenverarbeitungsmittel, die so konfiguriert sind, dass sie automatisch den Vergleich der Messung der CH4-Konzentration im zweiten Retentat mit einem Sollwert und die Einstellung des Drucks des Speisegasstroms durchführen.

2. Anlage nach Anspruch 1, **dadurch gekennzeichnet, dass** die in den Membrantrenneinheiten verwendeten Membranen die gleiche Selektivität aufweisen.

3. Verfahren zum Steuern einer Anlage, wie in einem der Ansprüche 1 oder 2 definiert, mit den folgenden Schritten:
- einen Schritt zum Messen des Saugdrucks des zweiten Permeats,
- einen Schritt des Vergleichens dieser Messung mit einem Sollwert und
- einen Schritt des Anpassens des Saugdrucks des zweiten Permeats durch den Kompressor B, um den Druckwert gleich dem Sollwert zu halten,
- einen Schritt zum Messen der CH4-Konzentration im zweiten Retentat,
- einen Schritt des Vergleichens dieser Messung mit einem Sollwert und des Bestimmens der Abweichung von diesem Sollwert, und
- einen Schritt zum Einstellen des Drucks des Speisegasstroms, um die ermittelte Abweichung zu verringern,
wobei die Einstellung des Drucks des Speisegasstroms mithilfe des Kompressors A oder mithilfe eines Ventils zur schrittweisen Abschaltung und Druckbeaufschlagung erfolgt.

4. Verfahren nach Anspruch 3, **dadurch gekennzeichnet, dass** der Kompressor B im Einstellungsschritt eine Beschleunigung oder eine Verzögerung erfährt.

5. Verfahren nach einem der Ansprüche 3 oder 4, **dadurch gekennzeichnet, dass** die Schritte des Messens des Saugdrucks des zweiten Permeats, des Vergleichens dieses Messwerts mit einem Sollwert und des Einstellens des Saugdrucks des zweiten Permeats automatisch durch Datenübertragungsmittel durchgeführt werden.

6. Verfahren nach einem der Ansprüche 3 bis 5, **dadurch gekennzeichnet, dass** der Schritt des Anpassens des Speisegasstroms eine Erhöhung oder Verringerung des Drucks umfasst.

7. Verfahren nach einem der Ansprüche 3 bis 6, **dadurch gekennzeichnet, dass** der Schritt des Vergleichens der Messung der CH4-Konzentration im zweiten Retentat mit einem Sollwert und der Schritt des Anpassens des Drucks des Speisegasstroms automatisch durch Datenübertragungs- und Datenverarbeitungsmittel durchgeführt werden.

8. Verfahren nach einem der Ansprüche 3 bis 7, **dadurch gekennzeichnet, dass** der Zufuhrgasstrom Biogas ist.

## Claims

1. Installation for treating a feed gas stream comprising at least methane and carbon dioxide by membrane permeation, comprising:
- a compressor A for compressing the feed gas stream,
- a first membrane separation unit adapted to receive the gas stream from the compressor and to provide a first permeate and a first retentate,
- a second membrane separation unit adapted to receive the first retentate and to provide a second permeate and a second retentate, the second permeate being configured to be recycled into the feed gas stream downstream of compressor A,
- a third membrane separation unit adapted to receive the first permeate and to provide a third permeate and a third retentate, the third retentate being configured to be recycled to compressor A for compressing the feed gas stream,
with each membrane separation unit comprising at least one membrane that is more permeable to carbon dioxide than to methane,
- at least one means for measuring the suction pressure of the second permeate from the second membrane unit,
- at least one compressor B for drawing the second permeate and adjusting the suction pressure of the second permeate based on the measured suction pressure before recycling the second permeate into the feed gas stream downstream of compressor A,
- data transmission means configured to automatically perform the measurement of the suction pressure of the second permeate, the comparison of this measurement with a setpoint value and the adjustment of the suction pressure of the second permeate by compressor B,
- at least one means for measuring the CH4 concentration in the second retentate,
- at least one means for adjusting the pressure of the feed gas stream based on the measured CH4 concentration,
the means for adjusting the pressure of the feed gas stream being compressor A or a shut-off and progressive pressurisation valve, and
- data transmission and data processing means configured to automatically perform the comparison of the measured CH4 concentration in the second retentate with a setpoint value and the adjustment of the pressure of the feed gas stream.

2. Installation according to claim 1, **characterised in that** the membranes used in the membrane separation units have the same selectivity.

3. Process for controlling an installation as defined in one of claims 1 or 2, comprising the following steps:
- a step of measuring the suction pressure of the second permeate,
- a step of comparing this measurement with a setpoint value, and
- a step of adjusting the suction pressure of the second permeate by compressor B to maintain the pressure value equal to the setpoint value,
- a step of measuring the CH4 concentration in the second retentate,
- a step of comparing this measurement with a setpoint value, and determining the deviation from this setpoint value, and
- a step of adjusting the pressure of the feed gas stream so as to reduce the determined deviation,
the adjustment of the pressure of the feed gas stream being carried out using compressor A or using a shut-off and progressive pressurisation valve.

4. A process according to claim 3, **characterised in that** in the adjustment step, compressor B undergoes acceleration or deceleration.

5. Process according to one of claims 3 or 4, **characterised in that** the steps of measuring the suction pressure of the second permeate, comparing this measurement with a setpoint value and adjusting the suction pressure of the second permeate are carried out automatically by data transmission means.

6. Process according to any one of claims 3 to 5, **characterised in that** the step of adjusting the feed gas stream comprises an increase or a decrease in pressure.

7. Process according to any one of claims 3 to 6, **characterised in that** the step of comparing the measured CH4 concentration in the second retentate with a setpoint value and the step of adjusting the pressure of the feed gas stream are carried out automatically by data transmission and data processing means.

8. Process according to any one of claims 3 to 7, **characterised in that** the feed gas stream is biogas.
